# EUROPEAN PATENT SPECIFICATION

(11) **EP 4 108 265 B1**
(45) Date of publication and mention of the grant of the patent: **03.09.2025**
(21) Application number: 21756791.6
(22) Date of filing: 08.02.2021
(51) Int. Cl.: A61L 27/36, A61L 27/50, A61L 33/00

(54) **FUNCTIONALIZED BIOLOGICAL MATRIX MATERIAL, PREPARATION METHOD THEREFOR AND USE THEREOF**
FUNKTIONALISIERTES BIOLOGISCHES MATRIXMATERIAL, VERFAHREN ZU SEINER HERSTELLUNG UND SEINE VERWENDUNG
MATÉRIAU FONCTIONNALISÉ DE MATRICE BIOLOGIQUE, PROCÉDÉ DE PRÉPARATION ASSOCIÉ ET UTILISATION CORRESPONDANTE

(30) Priority: 17.02.2020 CN 202010096214; 17.02.2020 CN 202010097281
(43) Date of publication of application: 28.12.2022
(73) Proprietor: Venus MedTech (HangZhou), Inc., Hangzhou, Zhejiang 310052 (CN)
(72) Inventor: WANG, Yunbing, Hangzhou, Zhejiang 310052 (CN); LEI, Yang, Hangzhou, Zhejiang 310052 (CN); JIN, Linhe, Hangzhou, Zhejiang 310052 (CN); GUO, Gaoyang, Hangzhou, Zhejiang 310052 (CN); YANG, Li, Hangzhou, Zhejiang 310052 (CN)
(74) Representative: KIPA AB
(86) International application number: PCT/CN2021/076114
(87) International publication number: WO 2021/164626

(56) References cited:
- WO-A1-2018/152444
- WO-A2-2018/155925
- CN-A- 101 945 676
- CN-A- 101 998 862
- CN-A- 111 166 938
- CN-A- 111 184 914
- US-A2- 2009 130 162
- GUO GAOYANG, JIN LINHE, JIN WANYU, CHEN LIANG, LEI YANG, WANG YUNBING: "Radical polymerization-crosslinking method for improving extracellular matrix stability in bioprosthetic heart vavles with reduced potential for calcification and inflammatory reponse", ACTA BIOMATERIALIA, vol. 82, 1 December 2018 (2018-12-01), pages 44 - 45, XP055839486, ISSN: 1742-7061, DOI: 10.1016/j.actbio.2018.10.017
- "Master Thesis", 28 March 2014, NANJING NORMAL UNIVERSITY, CN, article LUKIN: "The Effect of Different Material Surface-Modified Structures on Blood Compatibility Thereof)", pages: 1 - 69, XP009538675

## Description

### TECHNICAL FIELD

The present application relates to the technical field of medical materials, in particular to functionalized biological matrix materials and preparation methods and use thereof.

### BACKGROUND

Known commercial biological heart valves are generally prepared from bovine pericardium, porcine heart valve and porcine pericardium by crosslinking through glutaraldehyde. The crosslinking method through glutaraldehyde has the advantages of simple process, low cost, stable collagen structure and good mechanical properties. However, the cross-linked heart valves through glutaraldehyde have problems that cannot be ignored. The elastin cannot be protected in the case of crosslinking by glutaraldehyde, so the mechanical properties of the heart valve are easy to decline; the cross-linked heart valve through glutaraldehyde is easy to have residual and calcification problems, affecting the service life (< 10 years); glutaraldehyde and residual aldehyde groups therefrom are highly toxic and can cause inflammatory reactions, leading to valve failure.

In order to overcome the disadvantages of the cross-linked biological heart valves through glutaraldehyde, many methods have been used to improve or replace glutaraldehyde crosslinking. For example, after crosslinking through glutaraldehyde, the aldehyde group is blocked with a reagent such as diphosphate, α-aminooleic acid, and ethanol, but the effect is not significant. Further, some new cross-linking methods have been used for the crosslinking of biological heart valves, such as by carbodiimide, polyepoxy and genipin. Carbodiimide promotes the formation of amide bond from the reaction of amino group and carboxyl group of the protein through nucleophilic substitution, which does not introduce toxic aldehyde groups. However, since only adjacent amino and carboxyl groups can be cross-linked, the crosslinking effect is poor. The polyepoxy compound reacts with amino group, carboxyl group and hydroxyl group and the like through the epoxy group thereof, thereby achieving protein crosslinking. However, the stability of crosslinks is poor because the chemical bonds are prone to hydrolyze. The toxicity of genipin is less than that of glutaraldehyde, but the long-term stability and mechanical properties of the bioprosthetic heart valve cross-linked by genipin are inferior to those cross-linked by glutaraldehyde, and the bioprosthetic heart valve treated by genipin turns blue, and thus genipin is not widely used, either.

Further, the existing transcatheter biological valve products generally need to be immersed in glutaraldehyde solution for long-term preservation, so the products need to be washed to remove the residual glutaraldehyde before operation, and then be loaded into the valve delivery system in operation. This not only leads to the problem of glutaraldehyde residue, but also leads to the complicated procedure of before operation and the increased risk of surgical attachments. G.Guo et al. (Acta Biomaterialia 82,44-55 (2018)) disclosed a radical polymerization-crosslinking method for improving extracellular matrix stability in bioprosthetic heart valves with reduced potential for calcification and inflammatory response, wherein decellularized PP was first functionalized with the vinyl groups through the reaction with methacrylic anhydride (MA) and then crosslinked through a redox-initiating process. Chinese patent application No. CN202010096214.9 disclosed a functional acellular matrix biological material, and a preparation method thereof. According to the method, the acellular matrix biological material was hybridized with 3-sulfopropyl methacrylate, and crosslinking and functionalization of the acellular matrix biological material were simultaneously realized.

### SUMMARY

In order to overcome the disadvantages existing in the prior art, the present application provides a functionalized biological matrix material, a preparation method therefor and use thereof, which can replace the traditional crosslinking method of glutaraldehyde, and introduce 3-sulfopropyl methacrylate to provide the biological matrix material with better biocompatibility.

A method for preparing a functionalized biological matrix material, comprising steps of:
Cleaning a biological matrix material, immersing the biological matrix material in glycidyl methacrylate or methacrylic anhydride aqueous solution, and then in 3-sulfopropyl methacrylate aqueous solution, and adding an initiator to initiate polymerization, to realize the crossinking and functionalization of the biological matrix material; wherein the biological matrix material is an animal tissue subjected to decellularized treatment.

In the present application, a bifunctional amphiphilic molecule 3-sulfopropyl methacrylate containing a sulfonic acid group and an unsaturated double-bond functional group is introduced into the biological matrix material, which can form a polymer network in the collagen network of the biological matrix material, and provide the biological matrix material with better biocompatibility. More importantly, the sulfonic acid group can improve the hydrophilicity, so that the dry film being released can quickly absorb water and recover to be flattened in the human environment.

The steps for preparing the biological matrix material present a specific sequence and internally cooperate with each other, in which the biological matrix material is immersed in the glycidyl methacrylate or methacrylic anhydride aqueous solution, so as to introduce carbon-carbon double-bond structure, and then immersed in the 3-sulfopropyl methacrylate aqueous solution, so as to allow the 3-sulfopropyl methacrylate to be distributed between the protein fibers of the biological matrix material by means of physical permeation, and finally, the initiator is added to initiate polymerization and crosslinking of the double bond.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

The biological matrix material is an animal tissue obtained from swine, ovine or bovine, and the animal tissue comprises a blood vessel, heart valve and pericardium, and the main constituent thereof is at least one of collagen, elastin and glycosaminoglycan.

A decellularization agent can be added to the animal tissues for decellularization treatment to ensure that the cells are removed cleanly. Then, a cleaning liquid can be used to clean the biological matrix material by removing impurities such as residual cells and decellularization; wherein the cleaning liquid can be water, ethanol, acetone, etc.

Further, the methacrylic anhydride aqueous solution has a concentration of 1-20 wt%, preferably 1-5% wt%.

Further, the cleaned biological matrix material is immersed the methacrylic anhydride aqueous solution at 4-60 °C for 6-72 h, preferably, in the methacrylic anhydride aqueous at 20-40 °C for 18-24 h.

The cleaned biological matrix material reacts with the methacrylic anhydride to form amide bond through the reaction of amino group in the biological matrix material with methacrylic anhydride. In case of a low concentration of the methacrylic anhydride solution, a low temperature and a short time, the reaction between the biological matrix material and the methacrylic anhydride will be incomplete, the density of crosslinks will be low and the crosslinking of the biological matrix material will be insufficient, affecting the performance of the biological matrix material. However, a too high temperature will result in the destruction of the structure of the biological matrix material, and a high concentration of the methacrylic anhydride and a long time will cause unnecessary cost waste. Under the conditions of the temperature, time and concentration of the methacrylic anhydride solution according to the present invention, the biological matrix material and the methacrylic anhydride aqueous solution can be shaken to fully contact with each other, so that the methacrylic anhydride can fully react with the amino residue in the biological matrix material, increasing the connected double bond and the density of crosslinks, thereby providing a more stable cross-linked biological matrix material.

Further, the 3-sulfopropyl methacrylate aqueous solution has a concentration of 10-500 mmol / L.

Further, the biological matrix material is immersed in the 3-sulfopropyl methacrylate aqueous solution at 4-60 °C for 2-72 h, preferably, in the 3-sulfopropyl methacrylate aqueous solution at 20-45 °C for 18-36 h.

Before being immersed in the 3-sulfopropyl methacrylate aqueous solution, the biological matrix material is cleaned by using a cleaning agent, such as a mixture of ethanol and water in equal volumes, to clean up the unreacted free methacrylic anhydride. In the immersion process, the biological matrix material and the 3-sulfopropyl methacrylate aqueous solution can be shaken to fully contact with each other to ensure uniform dispersion of the 3-sulfopropyl methacrylate.

Further, the initiator is a photoinitiator or a thermal initiator; and wherein the photoinitiator is at least one of potassium persulfate, sodium persulfate and ammonium persulfate, and the thermal initiator is at least one of 2-hydroxy-2-methyl-1-phenylacetone, 1-hydroxycyclohexyl phenyl ketone, 2-methyl-2- (4-morpholinyl) -1 - [4-(methylthio) phenyl] -1-acetone, 2-hydroxy-4' - (2-hydroxyethoxy) -2-methylphenylacetone, 2,4,6-trimethylbenzoyl-diphenylphosphine oxide and 2-hydroxy-2-methyl-1-[4-(2-hydroxyethoxy) phenyl] -1-acetone.

Further, when the thermal initiator is used, the polymerization is performed at 20-45 °C for 18-48 h, and when the photoinitiator is used, the polymerization is performed at room temperature for 5-30 min under an ultraviolet light source.

The polymerization is a radical polymerization, in which the monomer concentration, the reaction time, and the reaction temperature are defined within a specified range so that the polymerization reaction can take place with a great degree of polymerization and an improved density of crosslinks, increasing the stability of the structure of the biological matrix material. If the respective condition is lower than the specified range, the reaction will be incomplete and the concentration of the connected monomer will be low, with a poor effect. If the reaction temperature is too high, the structure of the biological matrix material will be destroyed, and if the monomer concentration and reaction time are too high, a waste of cost will be resulted.

Further, the preparation method further comprises performing the following steps after initiating the polymerization reaction:
Crosslinking and fixing the obtained material from the polymerization by using a mixed solution of carbodiimide and N-hydroxysuccinimide; and
Immersing the obtained material from the crosslinking and fixing in a polyphenol solution.

Further, the material immersed in the polyphenol solution is rinsed, and stored with an antimicrobial solvent, or stored after dehydration and drying with an alcohol solution.

Further, the alcohol solution is a mixed solution of glycerol and ethanol or a mixed solution of glycerol, ethanol and isopropano.

Further, the alcohol solution is a mixed solution of 10-30 vt% glycerol and 70-90 vt% ethanol or a mixed solution of 10-30 vt% glycerol, 35-45 vt% ethanol and 35-45 vt% isopropanol.

Further, the step of adding an initiator to initiate polymerization comprises the steps of:
Thermally initiating double-bond polymerization at 20-45 °C for 12-48h, and wherein the initiator is at least one of potassium persulfate, ammonium persulfate, sodium bisulfite and tetramethylethylenediamine.

In addition, that step of immersing the obtained material from the crosslinking and fixing in a polyphenol solution comprises the following steps:
Immersing the obtained material from the crosslinking and fixing in 0.01-10 mM polyphenol compound aqueous solution for 1-24 hours, and wherein the polyphenol compound is at least one of curcumin, procyanidin, quercetin, resveratrol, aloin, aloe emodin, tannic acid, epigallocatechin gallate, pentagalloylglucose and genipin.

The present application further provides functionalized biological matrix materials prepared by any of the above methods.

The present application further provides the use of the functionalized biological matrix material in the preparation of a medical material. The medical material is a thoracotomy biological valve, an interventional biological valve, a tissue engineering heart valve, a biological patch, an artificial blood vessel or a tissue engineering blood vessel.

The functionalized biological matrix material has the following advantages over the prior art:
1. The present invention achieves multi-site and long-range crosslinking of a biological matrix material by means of a polymer network, and at the same time introduces corresponding functional functional groups so as to achieve functionalization of the biological matrix material. Specifically, the methacrylic anhydride fully reacts with the amino residues in the biological matrix material, modifying carbon-carbon double-bond structures such as allyl, methallyl in a biological matrix material, and then the biological matrix material is immersed in the aqueous solution containing 3-sulfopropyl methacrylate to get a better biocompatibility, and finally the biological matrix material is cross-linked and functionalized by means of radical polymerization. The hydrophilic property of the material is improved by introducing functional monomers having anticoagulant effect so as to effectively reduce the adhesion of platelets and improve the anticoagulant property of the biological matrix material. The material has various functions by introducing various substances through the method of the invention;
2. The density of crosslinks is improved by an interpenetrating network of the biomaterial prepared according to the method of the present invention with the polymer, thereby protecting the elastin, improving the mechanical properties of the biological matrix material and prolonging the service life thereof;
3. Glutaraldehyde is not introduced in the method of the present invention and most residues in the decellularized matrix are buried, so that the ability of the decellularized matrix material to bind calcium ion is reduced, thereby reducing the calcification reaction and improving the anti-calcification performance.

In order to solve the problems of residual aldehyde group of glutaraldehyde, easy calcification, failure to protect elastin and failure to be rapidly flattened of the folded dry valve material, the present application further provides a method for preparing a non-glutaraldehyde preloadable dry biological valve material using the functionalized biological matrix material and use thereof.

The present application further provides a method for preparing a non-glutaraldehyde preloadable dry biological valve material, comprising the steps of:
a, obtaining an animal pericardium material;
b, immersing the pericardium material in glycidyl methacrylate or acrylic anhydride solution to introduce carbon-carbon double-bond structure;
c, immersing the material treated in step b in a 3-sulfopropyl methacrylate aqueous solution;
d, adding an initiator to the material treated in step c for double-bond polymerization and crosslinking;
e, crosslinking and fixing the material obtained in step d by using a mixed solution of carbodiimide and N-hydroxysuccinimide;
f, immersing the material obtained in step e in a polyphenol solution; and
g rinsing the material treated in step f, storing the same with an antimicrobial solvent, or storing the same after dehydration and drying with an alcohol solution, thereby obtaining the non-glutaraldehyde preloadable dry biological valve material.

Carbodiimide promotes the formation of amide bond from the reaction of amino group and carboxyl group of the protein through nucleophilic substitution. However, the stability of crosslinks is poor because the chemical bonds are prone to hydrolyze. Therefore, a polyphenol crosslinking agent is further added. The pericardium material treated by the polyphenol crosslinking agent, based on hydrogen bonding, has a strong cross-link strength and stable components, without calcification after being implanted, and the mechanical strength thereof is equivalent to that treated by glutaraldehyde. The further crosslinking overcomes the defect of the instability of the crosslinks treated by carbodiimide, and improves the stability of the product. The biological valve material utilizes all reactive groups to increase the degree of crosslinking as much as possible to meet the requirements.

Steps a to d are essentially the same as the aforementioned method for preparing the functionalized biological matrix material, and the functionalized biological matrix material can also be treated by steps e to g, to obtain a preloadable dry biological valve material.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

Further, step a comprises decellularizing the animal pericardium material.

The biological valve material provided by the present application is a dry film, which can be directly pressed and loaded into the device in advance, and is always in a compressed state before use. In order to quickly flatten the valve after being released in the patient and avoid excessive wrinkles, the stability of elastin is improved by multi-step combined crosslinking (the content of amino group of elastin is low, and the stability of elastin is improved by promoting the carboxyl group, hydroxyl group and the like in crosslinking by the combined crosslinking), and 3-sulfopropyl methacrylate is introduced in step c to further improve the hydrophilicity, so that the dry film can be quickly recovered to be flattened by water absorption in the human environment. The steps for preparing the biological matrix material present a specific sequence and internally cooperate with each other, in which the biological matrix material is immersed in the glycidyl methacrylate or methacrylic anhydride aqueous solution, so as to introduce carbon-carbon double-bond structure, then immersed in the 3-sulfopropyl methacrylate aqueous solution by means of physical permeation, and then a polymerization is initiated together with the double bond introduced in the previous step b.

3-sulfopropyl methacrylate does not work independently, but co-works with the double bond introduced in step b through the double-bond structure thereof. Both the substances used in step b and step c need to have a C-C double bond, so that the double-bond polymerization can be carried out at one time in step d, and step b should be carried out before step c. Otherwise, if step c is carried out before step b, the immersion effect of the sulfonic acid group will be unsatisfied, and the protein fibers will be washed out in subsequent steps.

Further, the animal pericardium material in step a is obtained from swine, ovine or bovine.

Further, the decellularizing in step a comprises: immersing the pericardium in 0.01-1 wt% ethylenediaminetetraacetic acid disodium solution for 1-4 h, and then in 0.01-1 wt% sodium dodecyl sulfate solution for 1-24 h, and then rinsing the pericardium in sterile PBS solution for 1-24 h.

Preferably, the pericardium is immersed in 0.05-0.3 wt% ethylenediaminetetraacetic acid disodium solution for 1-2h, then in 0.05-0.3 wt% sodium dodecyl sulfate solution for 12-24h, and then rinsed in sterile PBS solution for 1-10h.

Further, step b specifically comprises: immersing the decellularized pericardium in 1-10 wt% glycidyl methacrylate solution for 3-7 days at 25-45°C or in 1-5 wt% methacrylic anhydride aqueous solution for 12-48 hours.

Further, step c specifically comprises: immersing the material in 0.01-1M 3-sulfopropyl methacrylate aqueous solution for 12-48h at 25-45 °C.

Preferably, the material is immersed in 0.05-0.5M 3-sulfopropyl methacrylate aqueous solution for 12-48 h at 30-40 °C

Further, step d specifically comprises: thermally initiating double-bond polymerization at 20-45 °C for 12-48h, and wherein the initiator is at least one of potassium persulfate, ammonium persulfate, sodium bisulfite and tetramethylethylenediamine.

Further, step e comprises: immersing the material treated by the double-bond polymerization and crosslinking in a pH buffer of 10-60 mM carbodiimide and 1-20 mM N-hydroxysuccinimide at 25-45 °C for 24-48 h.

Further, step f comprises: immersing the obtained material from the crosslinking and fixing in 0.01-10 mM polyphenol compound aqueous solution for 1-24 hours, and wherein the polyphenol compound is at least one of curcumin, procyanidin, quercetin, resveratrol, aloin, aloe emodin, tannic acid, epigallocatechin gallate, pentagalloylglucose and genipin.

Further, the material being stored with the antimicrobial solvent specifically includes: immersing the prepared biological valve material in 20-100 vt% isopropanol or 70-100 vt% ethanol aqueous solution for preservation. The material being stored after dehydration and drying with an alcohol solution specifically includes: immersing the prepared biological valve material in a mixed solution of 10-30vt% glycerol and 70-90vt% alcohol or a mixed solution of 10-30vt% glycerol, 35-45vt% ethanol and 35-45vt% isopropanol for 4-24 h, and drying.

The present application further discloses a non-glutaraldehyde preloadable dry biological valve material obtained by any of the above methods.

The present application further discloses use of the above non-glutaraldehyde preloadable dry biological valve material in preparation of biological valve and / or biological patch. The biological valve includes an artificial aortic valve, a pulmonary valve, a venous valve, a mitral valve, and a tricuspid valve.

In comparison with the prior art, the non-glutaraldehyde preloadable dry biological valve material, and the preparation method and use thereof have the following advantages:

As the crosslinking method according to the present invention does not involve glutaraldehyde, the problem of aldehyde groups of glutaraldehyde residual in the cross-linked pericardium by glutaraldehyde can be fundamentally avoided. The pericardium modified based on the glycidyl methacrylate or methacrylic anhydride aqueous solution involves double-bond polymerization and crosslinking, and the combined crosslinking by carbodiimide and polyphenol compound. The biological valve is developed as a dried interventional biological valve stored free of glutaraldehyde solution and pre-loaded into the valve delivery system. Compared to the cross-linked pericardium by glutaraldehyde, the biological valve according to the present invention has better anti-calcification properties and elastin stability, as well as rapid flattening properties after the dried valve material is folded, in order to solve the problems of short life of the existing biological valves due to easy calcification and failure to protect elastin, bringing a great significance for the research of biological heart valve and the development of related industrial applications.

### BRIEF DESCRIPTION OF THE DRAWINGS

In order to more clearly explain that technical solutions of the examples of the present invention, the drawings accompanying the examples will be briefly described. It can be understood that the following drawings show are only some examples of the present invention, and should not be construed as limiting the scope. Other related drawings can be obtained from these drawings without any creative effort by those skilled in the art.
Fig. 1 shows the weight loss rate by the degrading elastase;
Fig. 2 shows the calcium content;
Fig. 3 shows the relative adhesion rates of endothelial cells; and
Fig. 4 shows the results of the water immersion and flattening test.

### DESCRIPTION OF THE EMBODIMENTS

In order to make the objects, technical solutions and advantages of the present invention more clear and apparent, the present invention will be described in further detail with reference to the drawings and examples. It should be appreciated that the specific examples described herein are merely illustrative of the invention and are not intended to limit the invention. In other words, the described examples are only part of, but not all of, the examples of the invention. Generally, the components in the examples of the invention described and illustrated in the drawings herein can be arranged and designed in a variety of different configurations.

Therefore, the following detailed description of the examples of the invention referring to the drawings is not intended to limit the scope of the claimed invention, but is merely representative of selected examples of the invention. Based on the examples of the present invention, all other examples obtained by those skilled in the art without creative labor fall within the scope of protection of the present invention.

It should be noted that terms such as "the first" and "the second" are used only to distinguish one object or step from another object or step, without necessarily representing or implying any such relationship or order between these objects or steps in practice. Moreover, terms "comprising", "including", or the like, are intended to cover non-exclusive inclusions such that a process, method, object or apparatus comprising elements includes not only said elements, but also includes other elements that are not explicitly listed or are inherent to such process, method, object or apparatus. Without further limitation, an element defined by the statement "comprising a/an..." does not preclude a further identical element presented in the process, method, object or apparatus comprising said element.

The features and performances of the present invention are described in further detail below with reference to examples.

### Example 1

A method for preparing a functionalized biological matrix material provided by a preferred example of the present invention comprises the following steps:
1. Provide a fresh porcine pericardium and store it at 4 °C in wet;
2. Immerse the animal tissue material in 0. 1% EDTA solution for 1 hour, and then in 0. 1% SDS solution for 24 hours to perform decellularization treatment to prepare a decellularized matrix;
3. Clean the decellularized matrix by ultrasonic cleaning at room temperature with a cleaning solution prepared by distilled water and alcohol with a volume ratio of 1: 1;
4. Immerse the washed decellularized matrix in 1 wt% methacrylic anhydride aqueous solution at 25 °C for 24 hours;
5. Immerse the above material in 10 mmol / L 3-sulfopropyl methacrylate aqueous solution at 25 °C for 24 hours after ultrasonic cleaning at room temperature with the above cleaning solution; and
6. Add 5 mM potassium persulfate therein, reacting for 24 hours at 40 °C, and clean the material by ultrasonic cleaning with distilled water, thereby obtaining the functionalized biological matrix material.

### Example 2

A method for preparing a functionalized biological matrix material provided by a preferred example of the present invention comprises the following steps:
1. Provide a fresh porcine pericardium and store it at 4 °C in wet;
2. Immerse the animal tissue material in 0. 1% EDTA solution for 1 hour, and then in 0. 1% SDS solution for 24 hours to perform decellularization treatment to prepare a decellularized matrix;
3. Clean the decellularized matrix by ultrasonic cleaning at room temperature with a cleaning solution prepared by distilled water and alcohol with a volume ratio of 1: 1 ;
4. Immerse the washed decellularized matrix in 5 wt% methacrylic anhydride aqueous solution at 20 °C for 24 hours;
5. Immerse the above material in 50 mmol / L 3-sulfopropyl methacrylate aqueous solution at 30 °C for 24 hours after ultrasonic cleaning at room temperature with the above cleaning solution; and
6. Add 20 mM potassium persulfate therein, reacting for 24 hours at 37 °C, and clean the material by ultrasonic cleaning with distilled water, thereby obtaining the functionalized biological matrix material.

### Example 3

A method for preparing a functionalized biological matrix material provided by a preferred example of the present invention comprises the following steps:
1. Provide a fresh porcine pericardium and store it at 4 °C in wet;
2. Immerse the animal tissue material in 0. 1% EDTA solution for 1 hour, and then in 0. 1% SDS solution for 24 hours to perform decellularization treatment to prepare a decellularized matrix;
3. Clean the decellularized matrix by ultrasonic cleaning at room temperature with a cleaning solution prepared by distilled water and alcohol with a volume ratio of 1: 1 ;
4. Immerse the washed decellularized matrix in 3 wt% methacrylic anhydride aqueous solution at 30 °C for 24 hours;
5. Immerse the above material in 50 mmol / L 3-sulfopropyl methacrylate aqueous solution at 37 °C for 24 hours after ultrasonic cleaning at room temperature with the above cleaning solution; and
6. Add 20 mM potassium persulfate therein, reacting for 48 hours at 20 °C, and clean the material by ultrasonic cleaning with distilled water, thereby obtaining the functionalized biological matrix material.

### Example 4

A method for preparing a functionalized biological matrix material provided by a preferred example of the present invention comprises the following steps:
1. Provide a fresh porcine pericardium and store it at 4 °C in wet;
2. Immerse the animal tissue material in 0. 1% EDTA solution for 1 hour, and then in 0. 1% SDS solution for 24 hours to perform decellularization treatment to prepare a decellularized matrix;
3. Clean the decellularized matrix by ultrasonic cleaning at room temperature with a cleaning solution prepared by distilled water and alcohol with a volume ratio of 1: 1 ;
4. Immerse the washed decellularized matrix in 3 wt% methacrylic anhydride aqueous solution at 40 °C for 18 hours;
5. Immerse the above material in 200 mmol / L 3-sulfopropyl methacrylate aqueous solution at 40 °C for 18 hours after ultrasonic cleaning at room temperature with the above cleaning solution; and
6. Add Irgacure 2959 photoinitiator therein, irradiating for 10 min in an UV crosslink box, and clean the material by ultrasonic cleaning with distilled water, thereby obtaining the functionalized biological matrix material.

### Example 5

A method for preparing a functionalized biological matrix material provided by a preferred example of the present invention comprises the following steps:
1. Provide a fresh porcine pericardium and store it at 4 °C in wet;
2. Immerse the animal tissue material in 0. 1% EDTA solution for 1 hour, and then in 0. 1% SDS solution for 24 hours to perform decellularization treatment to prepare a decellularized matrix;
3. Clean the decellularized matrix by ultrasonic cleaning at room temperature with a cleaning solution prepared by distilled water and alcohol with a volume ratio of 1: 1;
4. Immerse the washed decellularized matrix in 1 wt% methacrylic anhydride aqueous solution at 40 °C for 18 hours;
5. Immerse the above material in 500 mmol / L 3-sulfopropyl methacrylate aqueous solution at 35 °C for 18 hours after ultrasonic cleaning at room temperature with the above cleaning solution; and
6. Add Irgacure 2959 photoinitiator therein, irradiating for 10 min in an UV crosslink box, and clean the material by ultrasonic cleaning with distilled water, thereby obtaining the functionalized biological matrix material.

### Example 6

A method for preparing a functionalized biological matrix material provided by a preferred example of the present invention comprises the following steps:
1. Provide a fresh porcine pericardium and store it at 4 °C in wet;
2. Clean the extracellular matrix by ultrasonic cleaning at room temperature with a cleaning solution prepared by distilled water and alcohol with a volume ratio of 1: 1;
3. Immerse the washed extracellular matrix in 2 wt% methacrylic anhydride aqueous solution at 30 °C for 24 hours;
4. Immerse the above material in 500 mmol / L 3-sulfopropyl methacrylate aqueous solution at 25 °C for 24 hours after ultrasonic cleaning at room temperature with the above cleaning solution;
6. Add 5 mM ammonium persulfate therein, reacting at 30 °C for 24 hours; and
7. Clean the material by ultrasonic cleaning with distilled water.

### Experimental Example 1

### Glutaraldehyde group samples:

The samples are prepared by treating decellularized biological matrix materials by traditional glutaraldehyde crosslinking, as glutaraldehyde valves for short: the porcine pericardium is immersed in 0.1 wt% ethylenediaminetetraacetic acid disodium solution for 1h, then in 0.1 wt% sodium dodecyl sulfate solution for 24h, rinsed in sterile PBS solution for 1h, and then successively immersed in 0.1 vt%, 0.5 vt%, 1 vt% glutaraldehyde PBS solution for crosslinking for 24h.

### Experimental group samples:

The materials prepared in Examples 1-3, respectively, as hybrid valves for short.

### Blank control group samples:

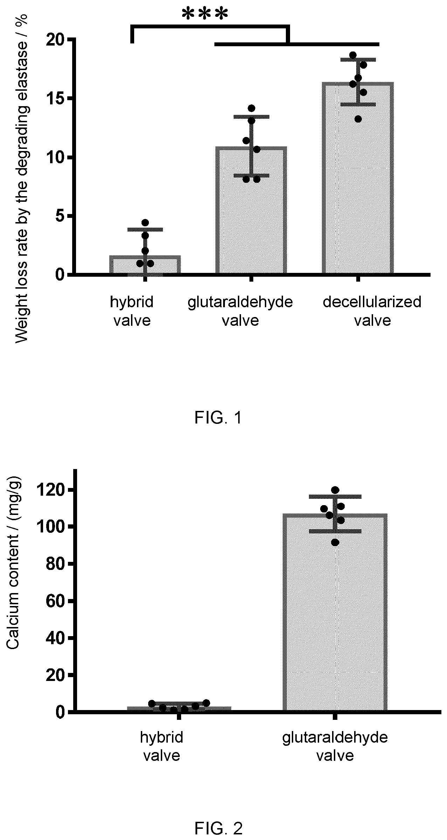

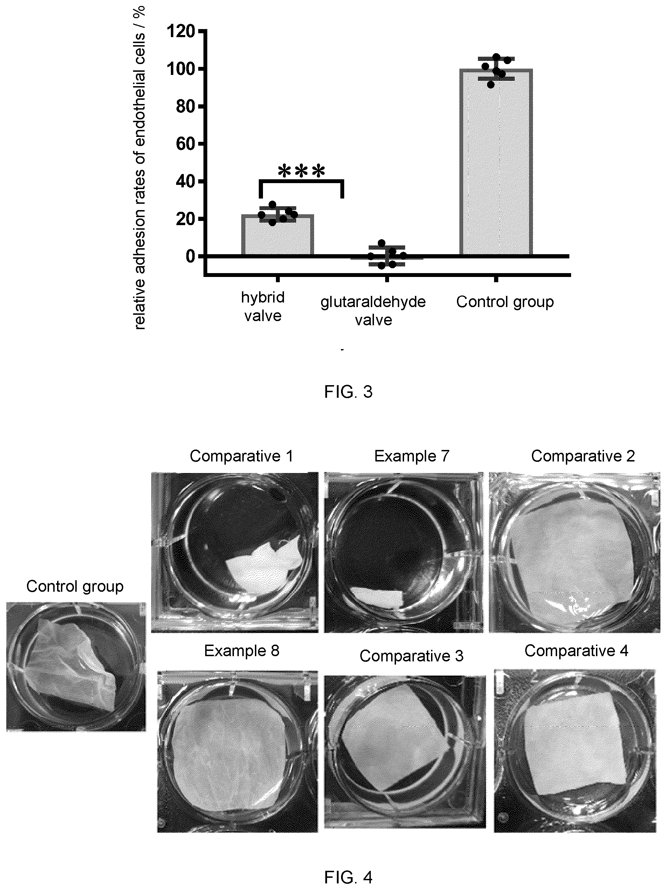

The decellularized biological matrix materials, as decellularized valves for short.
1. The weight losses of the glutaraldehyde group samples, the samples prepared in Example 1 and the blank control group samples after being degraded by elastase were measured, and the results are shown in FIG. 1.
   As can be seen from FIG. 1, the method in Example 1 can effectively protect elastin in the decellularized matrix, potentially improving the mechanical properties and prolonging the service life thereof. This is because the density of crosslinks is improved by an interpenetrating network of the biomaterial prepared according to the method of the present invention with the polymer, thereby protecting the elastin.
2. The calcium contents of the glutaraldehyde group samples and the samples prepared in Example 2 after subcutaneous implantation in rats for 30 days were measured, respectively, and the results are shown in FIG. 2.
   As can be seen from FIG. 2, the calcium contents of the materials prepared in Example 2 are far less than those of the glutaraldehyde group samples. Therefore, the method of the present invention can effectively reduce the calcification reaction. This is because glutaraldehyde is not introduce into the decellularized biological matrix material prepared according to the method of the present invention, and at the same time, most residues in the decellularized matrix are buried, so that the ability of the decellularized matrix material to bind calcium ion is reduced, thereby reducing the calcification reaction.
3. The relative adhesion rates of endothelial cells to the materials of the glutaraldehyde group samples, the samples prepared in Example 3 and the blank control group samples co-cultured with the endothelial cells for 1 day were measured, and the results are shown in FIG. 3.

As can be seen from FIG. 3, the method of the present invention can effectively improve the adhesion of endothelial cells on the decellularized biological matrix material. This is because the decellularized biological matrix material prepared according to the method of the present invention involves a biocompatible crosslinking agent, instead of a biotoxic crosslinking agent such as glutaraldehyde, and at the same time, functional monomers that promote the growth and migration of the endothelial cells are introduced to further improve the biocompatibility.

### Example 7

A method for preparing a non-glutaraldehyde preloadable dry biological valve material provided by a preferred example of the present invention comprises the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the decellularized pericardium material in 5 wt% glycidyl methacrylate solution and incubate at 37 °C for 3 days to introduce carbon-carbon double-bond structure.
c. Immerse the material obtained in the above step in 0.1 M 3-sulfopropyl methacrylate aqueous solution at 37 °C for 24 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 60 mM carbodiimide and 12 mM N-hydroxysuccinimide for crosslinking and fixation at 37 °C for 24 h.
f. Immerse the material obtained in the above step in 0.1 mM curcumin aqueous solution at room temperature for 24 hours, and rinse the material.
g. Immerse the rinsed material in a mixed solution of 20 vt% glycerol, 40 vt% ethanol and 40 vt% isopropanol for 4 h, followed by air dry, and store the material at room temperature.

### Example 8

A method for preparing a non-glutaraldehyde preloadable dry biological valve material provided by a preferred example of the present invention comprises the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the decellularized pericardium material in 2 wt% glycidyl methacrylate solution and incubate at 37 °C for 3 days to introduce carbon-carbon double-bond structure.
c. Immerse the material obtained in the above step in 0.1 M 3-sulfopropyl methacrylate aqueous solution at 37 °C for 24 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 60 mM carbodiimide and 12 mM N-hydroxysuccinimide for crosslinking and fixation at 37 °C for 24 h.
f. Immerse the material obtained in the above step in 0.1 mM curcumin aqueous solution at room temperature for 24 hours, and rinse the material.
g. Immerse the rinsed material in a mixed solution of 20 vt% glycerol, 40 vt% ethanol and 40 vt% isopropanol for 4 h, followed by air dry, and store the material at room temperature.

### Example 9

A method for preparing a non-glutaraldehyde preloadable dry biological valve material provided by a preferred example of the present invention comprises the following steps:
a. Immerse a porcine pericardium in 0.2 wt% ethylenediaminetetraacetic acid disodium solution for 1 h, then in 0.2 wt% sodium dodecyl sulfate solution for 20 h, and then rinsed in sterile PBS solution for 3 h.
b. Immerse the decellularized pericardium material in 8 wt% glycidyl methacrylate solution and incubate at 37 °C for 4 days to introduce carbon-carbon double-bond structure.
c. Immerse the material obtained in the above step in 0.2 M 3-sulfopropyl methacrylate aqueous solution at 37 °C for 24 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 50 mM carbodiimide and 15 mM N-hydroxysuccinimide for crosslinking and fixation at 37 °C for 24 h.
f. Immerse the material obtained in the above step in 1 mM procyanidin aqueous solution at room temperature for 24 hours, and rinse the material.
g. Immerse the rinsed material in a mixed solution of 20 vt% glycerol, 40 vt% ethanol and 40 vt% isopropanol for 4 h, followed by air dry, and store the material at room temperature.

### Example 10

A method for preparing a non-glutaraldehyde preloadable dry biological valve material provided by a preferred example of the present invention comprises the following steps:
a. Immerse a porcine pericardium in 0.3 wt% ethylenediaminetetraacetic acid disodium solution for 2 h, then in 0.3 wt% sodium dodecyl sulfate solution for 24 h, and then rinsed in sterile PBS solution for 5 h.
b. Immerse the decellularized pericardium material in 3 wt% methacrylic anhydride solution and incubate at 37 °C for 40 h to introduce carbon-carbon double-bond structure.
c. Immerse the material obtained in the above step in 0.5 M 3-sulfopropyl methacrylate aqueous solution at 37 °C for 20 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 60 mM carbodiimide and 12 mM N-hydroxysuccinimide for crosslinking and fixation at 37 °C for 30 h.
f. Immerse the material obtained in the above step in 0.5 mM resveratrol aqueous solution at room temperature for 20 hours, and rinse the material.
g. Immerse the rinsed material in a mixed solution of 20 vt% glycerol, 40 vt% ethanol and 40 vt% isopropanol for 4 h, followed by air dry, and store the material at room temperature.

### Example 11

A method for preparing a non-glutaraldehyde preloadable dry biological valve material provided by a preferred example of the present invention comprises the following steps:
a. Immerse the pericardium material in 10 wt% glycidyl methacrylate solution and incubate at 40 °C for 3 days to introduce carbon-carbon double-bond structure.
b. Immerse the material obtained in the above step in 0.5 M 3-sulfopropyl methacrylate aqueous solution at 37 °C for 24 h.
c. Add the material obtained in the above step into an aqueous solution of 20 mM ammonium persulfate and 20 mM sodium bisulfite for crosslinking at 37 °C for 24 hours.
d. Immerse the material obtained in the above step in a mixed solution of 60 mM carbodiimide and 12 mM N-hydroxysuccinimide for crosslinking and fixation at 37 °C for 24 h.
e. Immerse the material obtained in the above step in 1 mM curcumin aqueous solution at room temperature for 24 hours, and rinse the material.
f. Immerse the rinsed material in a mixed solution of 20 vt% glycerol, 40 vt% ethanol and 40 vt% isopropanol for 4 h, followed by air dry, and store the material at room temperature.

### Comparative 1

A biological valve material is prepared through the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the material obtained in the above step in 5 wt% glycidyl methacrylate solution and incubate at 37 °C for 3 days.
c. Immerse the material obtained in the above step in 0.1 M 3-sulfopropyl methacrylate aqueous solution at 37 °C for 24 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 60 mM carbodiimide and 12 mM N-hydroxysuccinimide for crosslinking and fixation at 37 °C for 24 h.
f. Rinse the material obtained in the above step and immerse the rinsed material in a mixed solution of 20 vt% glycerol, 40 vt% ethanol and 40 vt% isopropanol for 4 h, followed by air dry, and store the material at room temperature.

### Comparative 2

A biological valve material is prepared through the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the material obtained in the above step in 2 wt% methacrylic anhydride solution and incubate at 37 °C for 24 hours.
c. Immerse the material obtained in the above step in 0.1 M 3-sulfopropyl methacrylate aqueous solution at 37 °C for 24 h.
d. Add the material obtained in the above step into an aqueous solution of 20 mM ammonium persulfate and 20 mM sodium bisulfite for crosslinking at 37 °C for 24 hours.
e. Immerse the material obtained in the above step in a mixed solution of 60 mM carbodiimide and 12 mM N-hydroxysuccinimide for crosslinking and fixation at 37 °C for 24 h.
f. Rinse the material obtained in the above step and immerse the rinsed material in a mixed solution of 20 vt% glycerol, 40 vt% ethanol and 40 vt% isopropanol for 4 h, followed by air dry, and store the material at room temperature.

### Comparative 3

A biological valve material is prepared through the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the material obtained in the above step in a mixed solution of 60 mM carbodiimide and 12 mM N-hydroxysuccinimide for crosslinking and fixation at 37 °C for 24 h.
c. Rinse the material obtained in the above step and immerse the rinsed material in a mixed solution of 20 vt% glycerol, 40 vt% ethanol and 40 vt% isopropanol for 4 h, followed by air dry, and store the material at room temperature.

### Comparative 4

A biological valve material is prepared through the following steps:
a. Immerse a porcine pericardium in 0.1 wt% ethylenediaminetetraacetic acid disodium solution for 1 h, then in 0.1 wt% sodium dodecyl sulfate solution for 24 h, and then rinsed in sterile PBS solution for 1 h.
b. Immerse the material obtained in the above step in a mixed solution of 60 mM carbodiimide and 12 mM N-hydroxysuccinimide for crosslinking and fixation at 37 °C for 24 h.
c. Immerse the material obtained in the above step in 0.1 mM curcumin aqueous solution at room temperature for 24 hours.
d. Rinse the material obtained in the above step and immerse the rinsed material in a mixed solution of 20 vt% glycerol, 40 vt% ethanol and 40 vt% isopropanol for 4 h, followed by air dry, and store the material at room temperature.

### Experimental Example 2

A control group was provided and the samples thereof as well as the materials prepared in Examples 7 and 8 and Comparatives 1 to 4 were subjected to anti-calcification test, elastin stability test, and water immersion and flattening test, respectively.

In the control group: the porcine pericardium is immersed in 0.1 wt% ethylenediaminetetraacetic acid disodium solution for 1h, then in 0.1 wt% sodium dodecyl sulfate solution for 24h, rinsed in sterile PBS solution for 1h, and then successively immersed in 0.1 vt%, 0.5 vt%, 1 vt% glutaraldehyde PBS solution for crosslinking for 24h, then in 20 vt% glycerol, 40 vt% ethanol and 40 vt% isopropanol for 4 h, followed by air dry, and stored at room temperature.

### (1) Anti-calcification test

The samples from the example group, the comparative group and the control group were cut to the size of 1 cm * 1 cm and cleaned. 0.1 mL of 3% pentobarbital sodium was intraperitoneally injected into a young SD rat of about 20 days for anesthesia. Shave the fur off the muscles on both sides of the spine and disinfect with iodine and alcohol. One sample from the experimental group was implanted subcutaneously into the right back, and one sample from the control group was implanted subcutaneously into the left back, with the skin incisions sutured. After 60 days, the animals were euthanized by cervical dislocation and the implants were removed therefrom. Carefully removed the host tissue from the surface of the implants and rinse the implants with saline. Weigh the dried implant after freeze drying, and then digest with 6N concentrated hydrochloric acid in a water bath at 95 °C until no visible solid particles were present, followed by quantitative analysis of calcium using an inductively coupled plasma-optical emission spectrometer.

The materials obtained in the Example groups and the Control groups were subjected to anti-calcification test, and the results are shown in Table 1 below. As can be seen from Table 1, the calcium contents of the materials prepared in Example 7 and Example 8 are greatly reduced compared to the control group, and the calcium contents of the materials prepared in Example 7 and Example 8 are less than those of Comparatives 1 to 4. Therefore, the material of the present invention has excellent anti-calcification property.

**Table 1 Calcium Content**

| | Calcium content ( µ g / mg) |
|---|---|
| Control group | 19.41 ± 9.88 |
| Example 7 | 0.40 ± 0.05 |
| Example 8 | 0.44 ± 0.04 |
| Comparative 1 | 0.57 ± 0.31 |
| Comparative 2 | 0.56 ± 0.40 |
| Comparative 3 | 2.99 ± 2.32 |
| Comparative 4 | 2.24 ± 1.40 |

### (2) Elastin Stability Test

After the biological valve was implanted into the body, it will contact the protease in the blood. Under the action of the protease, the collagen and elastin in the valve will be degraded, thus affecting the stability of the biological valve. Enzymatic degradation experiment in vitro is an effective method to test the resistance of the biological valve to protease degradation. The component stability of the biological valve can be tested by simulating the protease environment in vivo. The biological valve, after freeze drying, was incubated in a Tris buffer(0.1 M Tris, 1 mM CaCl₂, pH = 7.8)containing elastase (30 U/mL) at 37 °C for 24 h, and then rinsed, and weighed after freeze drying. The dry weight loss rate was calculated.

The elastin stability results are shown in Table 2 below. As can be seen from Table 2 below, the weight loss rates by the degrading elastase of Examples 7 and 8 are greatly reduced relative to the control group, while the weight loss rates by the degrading elastase of Comparatives 2, 3 and 4 were only slightly reduced relative to the control group. In conclusion, the elastin stability of the material prepared by the invention is greatly improved.

**Table 2 Elastin Stability**

| | Weight loss rate by the degrading elastase (%) |
|---|---|
| Control group | 12.48 ± 0.44 |
| Example 7 | 6.36 ± 0.04 |
| Example 8 | 8.45 ± 0.49 |
| Comparative 1 | 6.48 ± 0.32 |
| Comparative 2 | 10.16 ± 0.33 |
| Comparative 3 | 11.16 ± 0.32 |
| Comparative 4 | 11.44 ± 0.36 |

### (3) Water immersion and flattening test

The materials prepared in Examples 7 and 8, Comparatives 1-4 and the control group were subjected to a folding and immersion test as follows: A plastic tube with an inner diameter of 5 mm was used for simulating a folding test; for each group, the materials were cut into a square sample with an area of about 3cm * 3cm with scissors; the square samples were slowly inserted into the plastic tube with an inner diameter of 5 mm by tweezers, and then placed in a constant temperature and humidity box at 40 °C and 60% -80% for 72 hours; the materials were then removed from the plastic tube and immersed in PBS buffer, and the immersion and flattening results were observed and photographed.

As shown in FIG. 4, both the examples and the control group have a good water immersion and flattening performance, while the control group (glutaraldehyde) could not become flattened quickly and the creases were clear.

## Claims

1. A method for preparing a functionalized biological matrix material, comprising steps of:
cleaning a biological matrix material,
immersing the biological matrix material in glycidyl methacrylate or methacrylic anhydride aqueous solution, and then in 3-sulfopropyl methacrylate aqueous solution, and
adding an initiator to initiate polymerization;
wherein the biological matrix material is an animal tissue subjected to decellularization treatment.

2. The method for preparing a functionalized biological matrix material according to claim 1, wherein the biological matrix material is an animal tissue obtained from swine, ovine or bovine, and the animal tissue comprises a blood vessel, heart valve and pericardium, and a main constituent thereof is at least one of collagen, elastin and glycosaminoglycan.

3. The method for preparing a functionalized biological matrix material according to claim 1 or 2, wherein the method has an additional feature a), or b), or c), or d):
a) the methacrylic anhydride aqueous solution has a concentration of 1-20 wt%;
b) the cleaned biological matrix material is immersed in the methacrylic anhydride aqueous solution at 4-60 °C for 6-72 h;
c) the 3-sulfopropyl methacrylate aqueous solution has a concentration of 10-500 mmol / L;
d) the biological matrix material is immersed in the 3-sulfopropyl methacrylate aqueous solution at 4-60 °C for 2-72 h.

4. The method for preparing a functionalized biological matrix material according to claim 1 or 2, wherein the initiator is a photoinitiator or a thermal initiator; and wherein the photoinitiator is at least one of potassium persulfate, sodium persulfate and ammonium persulfate, and the thermal initiator is at least one of 2-hydroxy-2-methyl-1-phenylacetone, 1-hydroxycyclohexyl phenyl ketone, 2-methyl-2- (4-morpholinyl) -1 - [4-(methylthio) phenyl] -1-acetone, 2-hydroxy-4' - (2-hydroxyethoxy) -2-methylphenylacetone, 2,4,6-trimethylbenzoyl-diphenylphosphine oxide and 2-hydroxy-2-methyl-1-[4-(2-hydroxyethoxy) phenyl] -1-acetone.

5. The method for preparing a functionalized biological matrix material according to claim 4, wherein when the thermal initiator is used, the polymerization is performed at 20-45 °C for 18-48 h, and when the photoinitiator is used, the polymerization is performed at room temperature for 5-30 min under an ultraviolet light source.

6. The method for preparing a functionalized biological matrix material according to claim 1, wherein the method further comprises, after initiating the polymerization:
crosslinking and fixing the obtained material from the polymerization by using a mixed solution of carbodiimide and N-hydroxysuccinimide; and
immersing the obtained material from the crosslinking and fixing in a polyphenol solution.

7. The method for preparing a functionalized biological matrix material according to claim 6, wherein the method has an additional feature a), or b):
a) the material immersed in the polyphenol solution is rinsed, and stored with an antimicrobial solvent, or stored after dehydration and drying with an alcohol solution;
b) the "immersing the obtained material from the crosslinking and fixing in a polyphenol solution" comprises:
immersing the obtained material from the crosslinking and fixing in 0.01-10 mM polyphenol compound aqueous solution for 1-24 hours, and wherein the polyphenol compound is at least one of curcumin, procyanidin, quercetin, resveratrol, aloin, aloe emodin, tannic acid, epigallocatechin gallate, pentagalloylglucose and genipin.

8. The method for preparing a functionalized biological matrix material according to claim 7 with feature a), wherein the method has an additional feature c), or d):
c) the alcohol solution is a mixed solution of glycerol and ethanol or a mixed solution of glycerol, ethanol and isopropanol;
d) the alcohol solution is a mixed solution of 10-30 vt% glycerol and 70-90 vt% ethanol or a mixed solution of 10-30 vt% glycerol, 35-45 vt% ethanol and 35-45 vt% isopropanol.

9. A functionalized biological matrix material prepared by the method according to any one of claims 1 to 8.

10. Use of the functionalized biological matrix material according to claim 9 in preparation of a medical material, wherein the medical material is a thoracotomy biological valve, an interventional biological valve, a tissue engineering heart valve, a biological patch, an artificial blood vessel or a tissue engineering blood vessel.

11. A method for preparing a non-glutaraldehyde preloadable dry biological valve material, comprising steps of:
a, obtaining an animal pericardium material;
b, immersing the pericardium material in glycidyl methacrylate or acrylic anhydride solution to introduce carbon-carbon double-bond structure;
c, immersing the material treated in step b in a 3-sulfopropyl methacrylate aqueous solution;
d, adding an initiator to the material treated in step c for double-bond polymerization and crosslinking;
e, crosslinking and fixing the material obtained in step d by using a mixed solution of carbodiimide and N-hydroxysuccinimide;
f, immersing the material obtained in step e in a polyphenol solution; and
g, rinsing the material treated in step f, storing the same with an antimicrobial solvent, or storing the same after dehydration and drying with an alcohol solution.

12. The method for preparing a non-glutaraldehyde preloadable dry biological valve material according to claim 11, wherein step a comprises decellularizing the animal pericardium material.

13. The method for preparing a non-glutaraldehyde preloadable dry biological valve material according to claim 11 or 12, wherein the method has an additional feature a), or b) , or c) , or d) , or e) , or f) , or g) , or h):
a) the animal pericardium material in step a is obtained from swine, ovine or bovine;
b) step b comprises: immersing the pericardium in 1-10 wt% glycidyl methacrylate solution for 3-7 days at 25-45°C or in 1-5 wt% methacrylic anhydride aqueous solution for 12-48 hours;
c) step c comprises: immersing the material in 0.01-1M 3-sulfopropyl methacrylate aqueous solution for 12-48h at 25-45 °C;
d) step d comprises: thermally initiating double-bond polymerization at 20-45 °C for 12-48h, and wherein the initiator is at least one of potassium persulfate, ammonium persulfate, sodium bisulfite and tetramethylethylenediamine;
e) step e comprises: immersing the material treated by the double-bond polymerization and crosslinking in a pH buffer of 10-60 mM carbodiimide and 1-20 mM N-hydroxysuccinimide at 25-45 °C for 24-48 h;
f) step f comprises: immersing the obtained material from the crosslinking and fixing in 0.01-10 mM polyphenol compound aqueous solution for 1-24 hours, and wherein the polyphenol compound is at least one of curcumin, procyanidin, quercetin, resveratrol, aloin, aloe emodin, tannic acid, epigallocatechin gallate, pentagalloylglucose and genipin;
g) the alcohol solution is a mixed solution of glycerol and ethanol or a mixed solution of glycerol, ethanol and isopropanol;
h) the alcohol solution is a mixed solution of 10-30 vt% glycerol and 70-90 vt% ethanol or a mixed solution of 10-30 vt% glycerol, 35-45 vt% ethanol and 35-45 vt% isopropanol.

14. A non-glutaraldehyde preloadable dry biological valve material prepared by the method according to any one of claims 11-13.

15. Use of the non-glutaraldehyde preloadable dry biological valve material according to claim 14 in preparation of biological valve and / or biological patch, wherein the biological valve comprises an artificial aortic valve, a pulmonary valve, a venous valve, a mitral valve, and a tricuspid valve.

## Patentansprüche

1. Ein System (100) zur Behandlung einer seitlichen Verkrümmung der Wirbelsäule, das Folgendes umfasst:
eine Vielzahl von Schrauben (102), die jeweils dafür vorgesehen sind, in einem entsprechenden Wirbelkörper einer Mehrzahl von Wirbelkörpern implantiert zu werden, wobei jede Schraube (102) einen Schraubenkopf (104) aufweist;
mehrere Verlängerungen (110), die jeweils dafür vorgesehen sind, lösbar mit einer der Schrauben (102) verbunden zu werden, wobei jede Verlängerungen (110) einen proximalen Endabschnitt (110a), einen distalen Endabschnitt (110b), der dafür vorgesehen ist, lösbar mit dem Schraubenkopf (104) einer entsprechenden Schraube (102) verbunden zu werden, und einen Mittelabschnitt zwischen dem proximalen Endabschnitt (110a) und dem distalen Endabschnitt (110b) aufweist; und
ein Stäbchen (130), die dafür vorgesehen ist, mit den Schraubenköpfen (104) verbunden zu werden;
wobei:
eine axiale Mittellinie des proximalen Endabschnitts (110a) von mindestens einer der Verlängerungen (110) in einem anderen Winkel liegt als eine axiale Mittellinie des distalen Endabschnitts (110b) der mindestens einen Verlängerung (110);
die Verlängerungen eine erste Verlängerung (110) und eine zweite Verlängerung (110) aufweisen; **mit der Eigenschaft, dass:**
eine axiale Mittellinie des proximalen Endabschnitts (110a) der ersten Verlängerung (110) in einem ersten Winkel bezüglich einer axialen Mittellinie des distalen Endabschnitts (110b) der ersten Verlängerung (110) liegt, wobei der erste Winkel größer als null ist;
eine axiale Mittellinie des proximalen Endabschnitts (110a) der zweiten Verlängerung in einem zweiten Winkel bezüglich einer axialen Mittellinie des distalen Endabschnitts (110b) der zweiten Verlängerung liegt,
wobei der zweite Winkel größer als der erste Winkel ist;
das System (100) so ausgelegt ist, dass das Stäbchen (130) entlang der mehreren Verlängerungen (110) von den proximalen Enden der mehreren Verlängerungen (110) in Richtung der distalen Endabschnitte (110b) der mehreren Verlängerungen (110) geführt und mit den Schrauben (102) in Verbindung gebracht werden kann.

2. Das System (100) nach Anspruch 1, wobei mindestens der Mittelabschnitt der mehreren Verlängerungen (110) gekrümmt ist.

3. Das System (100) nach Anspruch 1, wobei mindestens der Mittelabschnitt der mehreren Verlängerungen (110) gebogen ist.

4. Das System (100) nach einem der vorhergehenden Ansprüche, wobei der Winkel der axialen Mittellinie des proximalen Endabschnitts (110a) bezüglich der axialen Mittellinie des distalen Endabschnitts (110b) von mindestens einer der Verlängerungen (110) während eines Eingriffs zur Behandlung einer seitlichen Verkrümmung der Wirbelsäule von einem Chirurgen angepasst werden kann.

5. Das System (100) nach einem der vorhergehenden Ansprüche mit einer dritten Verlängerung, wobei eine axiale Mittellinie des proximalen Endabschnitts (110a) der dritten Verlängerung in einem dritten Winkel bezüglich einer axialen Mittellinie des distalen Endabschnitts (110b) der dritten Verlängerung liegt, wobei der dritte Winkel größer als der zweite Winkel ist.

6. Das System (100) nach Anspruch 5 mit einer vierten und einer fünften Verlängerung, wobei
eine axiale Mittellinie des proximalen Endabschnitts (110a) der vierten Verlängerung in einem vierten Winkel bezüglich einer axialen Mittellinie des distalen Endabschnitts (110b) der vierten Verlängerung liegt
der vierte Winkel größer als der dritte Winkel ist;
eine axiale Mittellinie des proximalen Endabschnitts (110a) der fünften Verlängerung in einem fünften Winkel bezüglich einer axialen Mittellinie des distalen Endabschnitts (110b) der fünften Verlängerung liegt; und
der fünfte Winkel größer als der vierte Winkel ist.

7. Das System (100) nach einem der vorhergehenden Ansprüche mit einer sechsten Verlängerung, wobei eine axiale Mittellinie des proximalen Endabschnitts (110a) der sechsten Verlängerung mit einer axialen Mittellinie des distalen Endabschnitts (110b) der sechsten Verlängerung kollinear ist.

8. Das System (100) nach einem der vorhergehenden Ansprüche, wobei es so ausgelegt ist, dass ein oder mehrere Wirbel in Richtung einer seitlichen Mittellinie der Wirbelsäule bewegt werden, wenn das Stäbchen (130) in Richtung der distalen Endabschnitte (110b) der mehreren Verlängerungen (110) vorgeschoben wird.

9. Das System (100) nach einem der vorhergehenden Ansprüche, wobei es so ausgelegt ist, dass das Stäbchen (130) gleichzeitig entlang der mehreren Verlängerungen (110) vorgeschoben werden kann, indem es schrittweise in Richtung des distalen Endabschnitts (110b) jeder der Verlängerungen (110) vorgeschoben wird.

10. Das System (100) nach einem der vorhergehenden Ansprüche, wobei das Stäbchen (130) zumindest in seitlicher Richtung im Wesentlichen gerade ist.

11. Das System (100) nach einem der vorhergehenden Ansprüche, wobei die mehreren Verlängerungen (110) unterschiedliche Längen aufweisen.

12. Das System (100) nach einem der vorhergehenden Ansprüche, wobei die mehreren Verlängerungen (110) unterschiedliche Krümmungen aufweisen, sodass sich eine Krümmung einer ersten der mehreren Verlängerungen (110) von einer Krümmung einer zweiten der mehreren Verlängerungen (110) unterscheidet.

13. Das System (100) nach einem der vorhergehenden Ansprüche, wobei jede der Verlängerungen (110) einen Schlitz (140) aufweist, der sich vom proximalen Endabschnitt (110a) in Richtung des distalen Endabschnitts (110b) jeder der Verlängerungen (110) erstreckt, wobei der Schlitz (140) so ausgelegt ist, dass er das Stäbchen (130) verschiebbar aufnimmt, sodass dieses durch die Schlitze (140) der Verlängerungen (110) in Richtung der Schraubenköpfe (104) geführt werden kann.

14. Das System (100) nach Anspruch 13, wobei der Schlitz (140) jeder der Verlängerungen (110) Innengewinde aufweist, die mit Gewindeschubelementen verschraubt werden, die in den Schlitzen (140) in Richtung der distalen Endabschnitte (1106) der mehreren Verlängerungen (110) vorgeschoben werden können, um das Stäbchen (130) in Richtung der Schraubenköpfe (104) vorzuschieben.

15. Das System (100) nach einem der vorhergehenden Ansprüche umfasst ferner mehrere Druckelemente, die mit den Verlängerungen (110) verbunden werden und das Stäbchen (130) selektiv in Richtung der distalen Endabschnitte (110b) der Verlängerungen (110) in Richtung der Schraubenköpfe (104) bewegen, wenn die Druckelemente in Richtung der distalen Endabschnitte (110b) der Verlängerungen (110) vorgeschoben werden.

## Revendications

1. Système (100) pour traiter une courbure latérale d'une colonne vertébrale, comprenant :
une pluralité de vis (102) chacune configurée pour être implantée dans une vertèbre correspondante parmi une pluralité de vertèbres, chaque vis de la pluralité de vis (102) présentant une tête de vis (104) ;
une pluralité d'extensions (110) chacune configurée pour être couplée de manière amovible à une vis correspondante parmi la pluralité de vis (102), chaque extension de la pluralité d'extensions (110) présentant une partie d'extrémité proximale (110a), une partie d'extrémité distale (110b) configurée pour couplée de manière amovible à la tête de vis (104) d'une vis correspondante (102), et une partie centrale entre la partie d'extrémité proximale (110a) et la partie d'extrémité distale (110b) ; et
une tige (130) qui est configurée pour être couplée à la pluralité de têtes de vis (104) ; où :
un axe axial de la partie d'extrémité proximale (110a) d'au moins une des extensions (110) est à un angle différent par rapport à un axe axial de la partie d'extrémité distale (110b) de la au moins une des extensions (110) ;
la pluralité d'extensions comprend une première extension (110) et une deuxième extension (110) ; **caractérisé en ce que**
un axe axial de la partie d'extrémité proximale (110a) de la première extension (110) est à un premier angle par rapport à un axe axial de la partie d'extrémité distale (110b) de la première extension (110), où le premier angle est supérieur à zéro ;
un axe axial de la partie d'extrémité proximale (110a) de la deuxième extension (110) est à un deuxième angle par rapport à un axe axial de la partie d'extrémité distale (110b) de la deuxième extension ;
le deuxième angle est supérieur au premier angle ;
le système (100) est configuré de telle sorte que la tige (130) peut être guidée le long de la pluralité d'extensions (110) depuis les extrémités proximales de la pluralité d'extensions (110) en direction des parties d'extrémité distales (110b) de la pluralité d'extensions (110) et en engagement avec la pluralité de vis (102).

2. Système (100) selon la revendication 1, dans lequel au moins la partie centrale de la pluralité d'extensions (110) est courbe.

3. Système (100) selon la revendication 1, dans lequel au moins la partie centrale de la pluralité d'extensions (110) est coudée.

4. Système (100) selon l'une quelconque des revendications précédentes, dans lequel l'angle de l'axe axial de la partie d'extrémité proximale (110a) par rapport à l'axe axial de la partie d'extrémité distale (110b) d'au moins une des extensions (110) peut être réglée par un chirurgien durant une procédure pour traiter une courbure latérale de la colonne vertébrale.

5. Système (100) selon l'une quelconque des revendications précédentes, comprenant une troisième extension, où un axe axial de la partie d'extrémité proximale (110a) de la troisième extension est à un troisième angle par rapport à un axe axial de la partie d'extrémité distale (110b) de la troisième extension, où le troisième angle est supérieur au deuxième angle.

6. Système (100) selon la revendication 5, comprenant une quatrième extension et une cinquième extension, où :
un axe axial de la partie d'extrémité proximale (110a) de la quatrième extension est à un quatrième angle par rapport à un axe axial de la partie d'extrémité distale (110b) de la quatrième extension ;
le quatrième angle est supérieur au troisième angle ;
un axe axial de la partie d'extrémité proximale (110a) de la cinquième extension est à un cinquième angle par rapport à un axe axial de la partie d'extrémité distale (110b) de la cinquième extension ; et
le cinquième angle est supérieur au quatrième angle.

7. Système (100) selon l'une quelconque des revendications précédentes, comprenant une sixième extension, où un axe axial de la partie d'extrémité proximale (110a) de la sixième extension est colinéaire à un axe axial de la partie d'extrémité distale (110b) de la sixième extension.

8. Système (100) selon l'une quelconque des revendications précédentes, où le système (100) est configuré pour déplacer une ou plusieurs vertèbres en direction d'un axe latéral de la colonne vertébrale à mesure que la tige (130) est avancée en direction des parties d'extrémité distales (110b) de la pluralité d'extensions (110).

9. Système (100) selon l'une quelconque des revendications précédentes, où le système (100) est configuré de telle sorte que la tige (130) peut être avancée simultanément vers le bas de la pluralité d'extensions (110) en avançant par incréments la tige (130) en direction de la partie d'extrémité distale (110b) de chaque extension de la pluralité d'extensions (110).

10. Système (100) selon l'une quelconque des revendications précédentes, dans lequel la tige (130) est généralement rectiligne au moins dans la direction latérale.

11. Système (100) selon l'une quelconque des revendications précédentes, dans lequel la pluralité d'extensions (110) ont des longueurs différentes.

12. Système (100) selon l'une quelconque des revendications précédentes, dans lequel la pluralité d'extensions (110) ont des courbures différentes de telle sorte qu'une courbure d'une première extension de la pluralité d'extensions (110) est différente d'une courbure d'une deuxième extension de la pluralité d'extensions (110).

13. Système (100) selon l'une quelconque des revendications précédentes, dans lequel chaque extension de la pluralité d'extensions (110) présente une fente (140) en son sein s'étendant depuis la partie d'extrémité proximale (110a) en direction de la partie d'extrémité distale (110b) de chaque extension de la pluralité d'extensions (110), où la fente (140) de chaque extension de la pluralité d'extensions (110) est configurée pour recevoir avec possibilité de coulissement la tige (130) en son sein de telle sorte que la tige (130) peut être guidée en direction de la pluralité de têtes de vis (104) à travers les fentes (140) de la pluralité d'extensions (110).

14. Système (100) selon la revendication 13, dans lequel la fente (140) de chaque extension de la pluralité d'extensions (110) présente des filets internes en son sein configurés pour s'engager par vissage avec une pluralité d'éléments de poussée filetés qui peuvent être avancés par vissage dans les fentes (140) en direction des parties d'extrémité distales (110b) de la pluralité d'extensions (110) pour amener la tige (130) à avancer en direction de la pluralité de têtes de vis (104).

15. Système (100) selon l'une quelconque des revendications précédentes, comprenant en outre une pluralité d'éléments de poussée configurés pour être couplés à la pluralité d'extensions (110) et pour déplacer de manière sélective la tige (130) vers le bas en direction des parties d'extrémité distales (110b) de la pluralité d'extensions (110) en direction de la pluralité de têtes de vis (104) à mesure que la pluralité d'éléments de poussée sont avancés en direction des parties d'extrémité distales (110b) de la pluralité d'extensions (110).
